Europäisches Patentamt

European Patent Office

Office européen des brevets

(19)

(11) **EP 0 962 237 A1**

(12) **EUROPÄISCHE PATENTANMELDUNG**

(43) Veröffentlichungstag:
08.12.1999 Patentblatt 1999/49

(51) Int. Cl.⁶: **A62D 3/00**

(21) Anmeldenummer: 99110327.6

(22) Anmeldetag: 27.05.1999

(84) Benannte Vertragsstaaten:
AT BE CH CY DE DK ES FI FR GB GR IE IT LI LU
MC NL PT SE
Benannte Erstreckungsstaaten:
AL LT LV MK RO SI

(30) Priorität: 27.05.1998 DE 19823556

(71) Anmelder: **Balle, Erika**
57220 Eblange (FR)

(72) Erfinder:
• **Däges, Johannes, Dr.**
66125 Dudweiler (DE)
• **Balle, Erika**
57220 Eblange (FR)

(74) Vertreter: **Wieske, Thilo et al**
Patentanwälte Vièl & Vièl,
Postfach 65 04 03
66143 Saarbrücken (DE)

(54) **Verfahren zur Entsorgung bzw. Verwertung von halogenierten Kohlenwasserstoffen**

(57)    Die Erfindung betrifft ein Verfahren zur Entsorgung von halogenierten Kohlenwasserstoffen, wobei den halogenierten Kohlenwasserstoffen bei der Entsorgung Energie zugeführt wird, um die halogenierten Kohlenwasserstoffe zu zerlegen, wobei die zugeführte Energie ein Intensitätsmaximum in dem Frequenzbereich aufweist, der dem Bereich der Schwingungsresonanzfequenz der jeweiligen Moleküle entspricht.

EP 0 962 237 A1

**Beschreibung**

Die vorliegende Erfindung betrifft ein Verfahren zur Entsorgung bzw. Verwertung von halogenierten Kohlenwasserstoffen, wobei den halogenierten Kohlenwasserstoffen Energie zugeführt wird, um die halogenierten Kohlenwasserstoffe zu zerlegen.

Dem Anmelder ist bereits ein derartiges Verfahren als Hochtemperaturverfahren bekannt, bei dem bei Temperaturen von etwa 2000°C mit vergleichsweise hohem technischen und energetischen Aufwand eine Zerlegung erfolgt. Durch nachgeschaltete Prozesse wird auch eine relativ hohe Rückführung der FCKW (HFCKW) Kältemittel in technisch verwertbare Stoffe wie beispielsweise Salzsäure, Flußsäure ermöglicht.

Es ist demgegenüber Aufgabe der vorliegenden Erfindung, ein Verfahren vorzuschlagen, mit dem insbesondere der energetische Aufwand zur Zerlegung der halogenierten Kohlenwasserstoffe minimiert werden soll.

Diese Aufgabe wird erfindungsgemäß durch ein Verfahren nach Anspruch 1 gelöst, wonach die zugeführte Energie ein Intensitätsmaximum in dem Frequenzbereich aufweist, der dem Bereich der Schwingungsresonanzfequenz der jeweiligen Moleküle entspricht.

Dieses Verfahren eignet sich insbesondere zur Zerlegung von fluorierten Kohlenwasserstoffen wie den vollhalogenierten Fluorchlorkohlenwasserstoffen (FCKW), wie beispielsweise R 11, R 12, R 13, R 113, R 114, R 115, den wasserstoffhaltigen teilhalogenierten Fluorchlorkohlenwasserstoffen (HFCKW), wie beispielsweise R22, R123, den perfluorierten Fluorkohlenwasserstoffen (FKW), wie beispielsweise R 14, R 116, R 218, oder den wasserstoffhaltigen teilhalogenierten Fluorkohlenwasserstoffen (HFKW), wie beispielsweise R 134a, R 227, R 125, R 32.

Bei der Energie handelt es sich um eine geeignete kurzwellige Strahlung im UV-Bereich. Die potentielle Energie der gegeneinander schwingenden Molekülteile wird dadurch selektiv angehoben bis die Dissoziationsenergie erreicht wird und damit die Bindung aufbricht.

Bei dem Verfahren nach Anspruch 2 weist die zugeführte Energie mehrere Intensitätsmaxima auf, die jeweils einem Frequenzbereich entsprechen, in dem eine Schwingungsresonanzfrequenz einer Bindung des Moleküls liegt.

Damit ist es möglich, die einzelnen Moleküle in mehrere Bestandteile zu zerlegen, indem Energie in Resonanz zu jeder aufzubrechenden Bindung zugeführt wird.

Bei der Ausgestaltung des Verfahrens nach Anspruch 3 werden dem halogenierten Kohlenwasserstoff nacheinander Energien mit jeweils einem Intensitätsmaximum zugeführt.

Dabei erweist es sich als vorteilhaft, daß definierte Bestandteile entstehen, indem die einzelnen Bindungen nacheinander aufgebrochen werden.

Bei der Ausgestaltung des Verfahrens nach Anspruch 4 wird dem halogenierten Kohlenwasserstoff vorab thermisch breitbandige Energie zugeführt.

Vorteilhaft stellt sich durch diese Energie eine Temperatur in der Größenordnung von einigen hundert Grad C ein.

Bei der Ausgestaltung des Verfahrens nach Anspruch 5 werden aus den zerlegten Bestandteilen des halogenierten Kohlenwasserstoffs mittels zugegebenen Bindungspartnern neue Stoffe erzeugt.

Die insbesondere bei der Zerlegung von FCKW entstehenden Halone Chlorid und Fluorid zählen zu den reaktivsten Elementen überhaupt. Sie bleiben nicht ohne Partner sondern suchen sich neue Bindungspartner bzw. rekombinieren wieder zu einem FCKW.

Es hat sich gezeigt, daß die zu diesem Zeitpunkt herrschenden physikalischen Bedingungen wie Druck und Temperatur und die als potentielle Bindungspartner nach der Zerlegung des halogenierten Kohlenwasserstoffs zur Verfügung stehenden Stoffe und Elemente die neu entstehenden Molekülkombinationen bestimmen.

Je nach Art des potentiellen Bindungspartners kann dieser mit dem zu zerlegenden halogenierten Kohlenwasserstoff eingespeist oder erst im Bereich der Bestrahlung zugeführt werden.

Bei der Ausgestaltung des Verfahrens nach Anspruch 6 werden die Bindungspartner zugegeben, indem wenigstens einem zugeführten Ausgangsstoff eine Energie zugeführt wird, die ein Intensitätsmaximum in dem Frequenzbereich aufweist, der dem Bereich der Schwingungsresonanzfequenz der jeweiligen Moleküle des Ausgangsstoffes entspricht.

Dadurch kann der potentielle Bindungspartner als Bestandteil eines Ausgangsstoffes zugeführt werden, der ebenfalls unter energetisch günstigen Bedingungen zerlegt wird. Der Bindungspartner kann daher durch eine entsprechende Beaufschlagung mit Energie auch im richtigen Zeitpunkt zur Verfügung gestellt werden.

Bei der Ausgestaltung des Verfahrens nach Anspruch 7 weist die dem wenigstens einen Ausgangsstoff zugeführte Energie mehrere Intensitätsmaxima auf, die jeweils einem Frequenzbereich entsprechen, in dem eine Schwingungsresonanzfrequenz einer Bindung des Moleküls des Ausgangsstoffes liegt.

Dadurch können die Bindungspartner auch aus Ausgangsstoffen mit einem komplizierteren Molekülaufbau mit energetisch verhältnismäßig geringem Aufwand herausgelöst werden.

Bei dem Verfahren nach Anspruch 8 werden dem Ausgangsstoff nacheinander Energien mit jeweils einem Intensitätsmaximum zugeführt.

Dadurch kann eine Zerlegung des Ausgangsstoffes in mehreren Schritten erfolgen bis der gewünschte Bindungspartner zur Verfügung steht.

Bei dem Verfahren nach Anspruch 9 wird dem Ausgangsstoff vorab thermisch breitbandige Energie zugeführt.

Dadurch wird vorteilhaft die Zerlegung des Ausgangsstoffes der Bindungspartner begünstigt vergleichbar den Verhältnissen wie dies bei der Zerlegung der halogenierten Kohlenwasserstoffe im Zusammenhang mit Anspruch 4 beschrieben wurde.

Bei dem Verfahren nach Anspruch 10 ist der zugeführte Ausgangsstoff Wasser, das in der dampfförmigen Phase zugeführt wird.

Als ein bevorzugter Bindungspartner für die Halone ist Wasserstoff anzusehen.

Bei Temperaturen über 200°C liegt Wasser in Form von Wasserdampf vor und kann in dieser Form den Bereich der Bestrahlung passieren. Bei Wellenlängen der zugeführten Energie von beispielsweise 170nm dissoziiert Wasserdampf unter günstigen energetischen Bedingungen zu Radikalen H, O und OH, die somit dann als Bindungspartner zur Verfügung stehen.

Bei dem Verfahren nach Anspruch 11 erfolgt die Reaktion der zerlegten Bestandteile des halogenierten Kohlenwasserstoffs mit den Bindungspartnern unter einem erhöhten Druck.

Es hat sich gezeigt, daß unter diesen Bedingungen bevorzugt höhermolekulare Verbindungen entstehen. Wird beispielsweise Trichlorfluormethan bei 300°C in Anwesenheit von Wasserdampf mit Licht der Wellenlängen von etwa 170nm bis 280nm bestrahlt, so entsteht bei Drücken von 2 bis 2,5 bar die Verbindung Tetrachlorkohlenstoff. Die Reaktionsgleichung lautet:

$$4CCl_3F + 2H_2O \rightarrow 3CCl_4 + CO_2 + 4HF$$

Bei der Ausgestaltung des Verfahrens nach Anspruch 12 erfolgt die Reaktion der zerlegten Bestandteile des halogenierten Kohlenwasserstoffs mit den Bindungspartnern in der gasförmigen Phase. Die gasförmigen Endstoffe werden separiert, indem das Gasgemisch abgekühlt wird und indem bei Unterschreiten der Siedetemperatur des Endproduktes dieses Endprodukt abgeschieden wird.

Durch ein langsames Abkühlen werden die Endstoffe aufgrund ihrer unterschiedlichen Siedepunkte nach und nach verflüssigt und können so getrennt werden. Wenn beispielsweise aus Trichlorfluormethan Tetrachlorkohlenstoff erzeugt wurde, verbleibt Tetrachlorkohlenstoff bei Abkühlung unter 76°C in der flüssigen Phase, während Trichlorfluormethan in der Gasphase verbleibt.

Bei der Ausgestaltung des Verfahrens nach Anspruch 13 werden nicht abgeschiedene Gase erneut mit Energie beaufschlagt.

Dadurch kann die Zerlegung der halogenierten Kohlenwasserstoffe und die Gewinnung von verwertbaren Endstoffen weiter verbessert werden. In dem im Zusammenhang mit Anspruch 12 erläuterten Ausführungsbeispiel kann beispielsweise das in der Gasphase verbliebene Trichlorfluormethan die Bestrahlungszone zum zweiten Mal durchlaufen.

Bei der Ausgestaltung des Verfahrens nach Anspruch 14 erfolgt die Reaktion der zerlegten Bestandteile des halogenierten Kohlenwasserstoffs mit den Bindungspartnern in der gasförmigen Phase. Die gasförmigen Endstoffe werden separiert, indem das Gasgemisch durch eine Waschlauge geleitet wird, in der die Endstoffe in Lösung gehen.

Auch dadurch können die Endstoffe von den Stoffen getrennt werden, die noch nicht wie gewünscht reagiert haben. Beispielsweise entstehen bei einem ausreichenden Wasserstoffangebot die Verbindungen HF und HCl. Diese gehen bei der Durchleitung durch eine Natronlauge in Lösung und verbleiben in der Natronlauge.

Bei dem Verfahren nach Anspruch 15 werden nicht in Lösung gegangene Gase erneut mit Energie beaufschlagt.

In dem im Zusammenhang mit dem vorhergehenden Anspruch erläuterten Ausführungsbeispiel können beispielsweise die noch nicht umgewandelten Gasanteile durch die Natronlauge durchgeleitet werden, wenn deren Temperatur oberhalb der Siedetemperatur der Gasanteile liegt. Diese Gase können dann erneut einer Bestrahlung zugeführt werden. Dadurch kann ebenfalls die Zerlegung der halogenierten Kohlenwasserstoffe und die Gewinnung der Endstoffe verbessert werden.

Es zeigt sich also, daß mit der vorliegenden Erfindung insbesondere durch die Kombination der Maßnahmen Resonanzbestrahlung, Angebot geeigneter Bindungspartner für die Molekülfragmente sowie die Selektion der entstandenen Endstoffe aufgrund von deren physikalischen oder chemischen Eigenschaften eine große Anzahl von halogenierten Kohlenwasserstoffen, insbesondere den fluorierten Kohlenwasserstoffen wie den eingangs erläuterten FCKW's, HFCKW's, FKW's, HFKW's, sowie auch HCKW's oder CKW's mit relativ geringem Energieeinsatz in andere, besser handhabbare Stofformen überführt werden kann.

## Patentansprüche

1. Verfahren zur Entsorgung bzw. Verwertung von halogenierten Kohlenwasserstoffen, wobei den halogenierten Kohlenwasserstoffen bei der Entsorgung bzw. Verwertung Energie zugeführt wird, um die halogenierten Kohlenwasserstoff zu zerlegen, dadurch gekennzeichnet, daß die zugeführte Energie ein Intensitätsmaximum in dem Frequenzbereich aufweist, der dem Bereich der Schwingungsresonanzfequenz der jeweiligen Moleküle entspricht.

2. Verfahren nach Anspruch 1, dadurch gekennzeichnet, daß die zugeführte Energie mehrere Intensitätsmaxima aufweist, die jeweils einem Frequenzbereich entsprechen, in dem eine Schwingungsresonanzfrequenz einer Bindung des Moleküls liegt.

3. Verfahren nach Anspruch 2,
dadurch gekennzeichnet, daß dem halogenierten Kohlenwasserstoff nacheinander Energien mit jeweils einem Intensitätsmaximum zugeführt werden.

4. Verfahren nach einem der Ansprüche 1 bis 3,
dadurch gekennzeichnet, daß dem halogenierten Kohlenwasserstoff vorab thermisch breitbandige Energie zugeführt wird.

5. Verfahren nach einem der Ansprüche 1 bis 4,
dadurch gekennzeichnet, daß aus den zerlegten Bestandteilen des halogenierten Kohlenwasserstoffs mittels zugegebenen Bindungspartnern neue Stoffe erzeugt werden.

6. Verfahren nach Anspruch 5,
dadurch gekennzeichnet, daß die Bindungspartner zugegeben werden, indem wenigstens einem zugeführten Ausgangsstoff eine Energie zugeführt wird, die ein Intensitätsmaximum in dem Frequenzbereich aufweist, der dem Bereich der Schwingungsresonanzfequenz der jeweiligen Moleküle des Ausgangsstoffes entspricht.

7. Verfahren nach Anspruch 6,
dadurch gekennzeichnet, daß die dem wenigstens einen Ausgangsstoff zugeführte Energie mehrere Intensitätsmaxima aufweist, die jeweils einem Frequenzbereich entsprechen, in dem eine Schwingungsresonanzfrequenz einer Bindung des Moleküls des Ausgangsstoffes liegt.

8. Verfahren nach Anspruch 7,
dadurch gekennzeichnet, daß dem Ausgangsstoff nacheinander Energien mit jeweils einem Intensitätsmaximum zugeführt werden.

9. Verfahren nach einem der Ansprüche 6 bis 8,
dadurch gekennzeichnet, daß dem Ausgangsstoff vorab thermisch breitbandige Energie zugeführt wird.

10. Verfahren nach Anspruch 9,
dadurch gekennzeichnet, daß der zugeführte Ausgangsstoff Wasser ist, das in der dampfförmigen Phase zugeführt wird.

11. Verfahren nach einem der Ansprüche 5 bis 10,
dadurch gekennzeichnet, daß die Reaktion der zerlegten Bestandteile des halogenierten Kohlenwasserstoffs mit den Bindungspartnern unter einem erhöhten Druck erfolgt.

12. Verfahren nach einem der Ansprüche 5 bis 11,
dadurch gekennzeichnet, daß die Reaktion der zerlegten Bestandteile des halogenierten Kohlenwasserstoffs mit den Bindungspartnern in der gasförmigen Phase erfolgt und daß die gasförmigen Endstoffe separiert werden, indem das Gasgemisch abgekühlt wird und indem bei Unterschreiten der Siedetemperatur des Endproduktes dieses Endprodukt abgeschieden wird.

13. Verfahren nach Anspruch 12,
dadurch gekennzeichnet, daß nicht abgeschiedene Gase erneut mit Energie beaufschlagt werden.

14. Verfahren nach einem der Ansprüche 5 bis 11,
dadurch gekennzeichnet, daß die Reaktion der zerlegten Bestandteile des halogenierten Kohlenwasserstoffs mit den Bindungspartnern in der gasförmigen Phase erfolgt und daß die gasförmigen Endstoffe separiert werden, indem das Gasgemisch durch eine Waschlauge geleitet wird, in der die Endstoffe in Lösung gehen.

15. Verfahren nach Anspruch 14,
dadurch gekennzeichnet, daß nicht in Lösung gegangene Gase erneut mit Energie beaufschlagt werden.

Europäisches
Patentamt

**EUROPÄISCHER RECHERCHENBERICHT**

Nummer der Anmeldung

EP 99 11 0327

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | EP 0 553 776 A (CONRAD SIEGFRIED DR ;SCHOENGARTH BERND (DE)) 4. August 1993 (1993-08-04) * Spalte 5, Zeile 15-27 * * Spalte 6, Zeile 1 - Spalte 7, Zeile 4 * * Spalte 10, Zeile 39 - Spalte 11, Zeile 8 * * Ansprüche 4-13,38-42 * --- | 1-15 | A62D3/00 |
| X | US 5 714 665 A (SAKAI HISASHI ET AL) 3. Februar 1998 (1998-02-03) * Spalte 4, Zeile 23-37 * * Spalte 4, Zeile 46 - Spalte 5, Zeile 5 * * Spalte 9, Zeile 45 - Spalte 10, Zeile 3 * * Ansprüche * --- | 1-4 | |
| X | DE 40 16 514 A (INT BIOTECH LAB) 28. November 1991 (1991-11-28) * Seite 2, Zeile 49-52 * * Ansprüche * --- | 1-8 | |
| X | DE 87 04 276 U (H: M. HARREB) 6. August 1987 (1987-08-06) * Seite 7; Ansprüche; Beispiel 1 * --- | 1 | RECHERCHIERTE SACHGEBIETE (Int.Cl.6)  A62D |
| A | ASKARYAN G A ET AL: "A FREELY LOCALIZED MICROWAVE DISCHARGE FOR REMOVAL OF CHLOROFLUOROCARBON CONTAMINATION FROM THE ATMOSPHERE" JOURNAL OF PHYSICS D. APPLIED PHYSICS, Bd. 27, Nr. 6, 14. Juni 1994 (1994-06-14), Seiten 1311-1318, XP000456959 ISSN: 0022-3727 ----- | | |

Der vorliegende Recherchenbericht wurde für alle Patentansprüche erstellt

| Recherchenort | Abschlußdatum der Recherche | Prüfer |
|---|---|---|
| DEN HAAG | 13. Juli 1999 | Dalkafouki, A |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
...............................................................................
& : Mitglied der gleichen Patentfamilie,übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 99 11 0327

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-07-1999

| Im Recherchenbericht angeführtes Patentdokument | | Datum der Veröffentlichung | Mitglied(er) der Patentfamilie | | Datum der Veröffentlichung |
|---|---|---|---|---|---|
| EP 0553776 | A | 04-08-1993 | DE | 4205161 A | 29-07-1993 |
| US 5714665 | A | 03-02-1998 | KEINE | | |
| DE 4016514 | A | 28-11-1991 | KEINE | | |
| DE 8704276 | U | 06-08-1987 | KEINE | | |

EPO FORM P0461

Für nähere Einzelheiten zu diesem Anhang : siehe Amtsblatt des Europäischen Patentamts, Nr.12/82